# EUROPEAN PATENT APPLICATION

(11) **EP 0 814 162 A2**
(43) Date of publication of application: **29.12.1997**
(21) Application number: 97109634.2
(22) Date of filing: 13.06.1997
(51) Int. Cl.: C12N 15/31, C12N 15/62, C12N 15/90, C12N 1/21

(54) **Expression of the alpha-toxin gene (hla) of staphylococcus aureus using a chromosomally encoded hla-lacZ gene fusion**

(30) Priority: 21.06.1996 EP 96110045
(71) Applicant: HOECHST AKTIENGESELLSCHAFT, 65929 Frankfurt am Main (DE)
(72) Inventor: Koller, Klaus-Peter, Dr., 65812 Bad Soden (DE); Ohlsen, Knut, Dr., 97080 Würzburg (DE); Hacker, Jörg, Prof. Dr., 97218 Gerbrunn (DE)

(57) **Abstract**

The present invention relates to a Staphylococcus aureus strain containing an reporter gene operatively linked to the promoter of the hla-gene, wherein the reporter gene is the lacZ gene; a bacterial strain, obtainable by transforming staphylococcus aureus wood 46 with hla-lacZ fusion gene using the recombinant plasmid pkO10 carrying said fusion gene, the bacterial strain Staphyloccocus aureus Wood 46-3; the use of a bacterial strain according to any of the proceeding claims to measure the hla promoter activity under different growth conditions, wherein the different growth conditions are selected from the group consisting of length of growth time and phase, temperature, osmolarity, glucose concentration, solid/liquid media, oxygen concentration, and concentration of low, subinhibitory concentrations of antibiotics; the vector pkO10, and a process for the production of Staphylococcus aureus 46-3.

## Description

*Staphylococcus aureus* is an important pathogenic bacterium, which causes a variety of human infectious diseases, including endocarditis, osteomyelitis, dermonecrosis, skin abcesses and pneumonia (49, 68, 70). The organism produces a large number of extracellular and cell associated proteins, many of which are involved in pathogenesis, such as alpha-, beta-, gamma-, delta-toxin, leukocidin, toxic shock syndrome toxin (TSST), enterotoxins, coagulase, protein A, fibrinogen-, fibronectin binding protein and others (2, 32, 34). The alpha-toxin (*hla*) is one of the major virulence factors of *Staphylococcus aureus* and is produced by the majority of the strains (44). The protein is a pore forming toxin and has cytolytic, hemolytic, dermonecrotic and lethal activities. A wide range of celltypes is affected by α-toxin, including erythrocytes, monocytes, lymphocytes, macrophages, epithelial cells, fibroblasts and kera- tinocytes (7, 8, 9, 69).

Staphylococcal α-toxin is secreted as a water-soluble single chain polypeptide of 34 kDa (8). There are two ways how α-toxin interacts with target cells; (i) it can bind to specific, high affinity receptors of unknown structure on the cell surface of human platelets, monocytes, endothelial cells, rabbit erythrocytes and others or (ii) it can interact unspecifically by ad-sorption to lipid bilayers. In both cases, six molecules aggregate to form a hexameric trans- membrane channel of 1-2 nm diameter. Subsequent efflux of K⁺ and nucleotides and influx of Na⁺ and Ca²⁺ leads to osmotic lysis or multiple secondary actions, including eicosanoid production, secretory processes, contractile dysfunction, apoptosis and release of cytokins (8, 15, 31, 63). Although much has been learned about the α-toxin action on a molecular level, little is known about the processes occuring during staphylococcal infections of α-toxin producing strains in humans. Direct evidences for the pathological importance of the toxin came from studies with site-specific *hla* negative mutants of *S. aureus* in mouse infection models. It has been shown that *hla*-negative mutants of *S. aureus* are significantly less virulent than the corresponding wild type strains (11, 51, 52). Further it has been demonstrated that specific antibodies contribute to protection of toxic effects *in vitro* and *in vivo* (8).

Like most staphylococcal exoproteins, α-toxin is not expressed constitutively, but rather its production is regulated by an array of extracellular and intracellular signals. The bacteria are able to sense surrounding environmental signals such as temperature, pH, nutrition, oxygen, osmolarity, CO₂, host factors and others, and respond by activating specific genes. A complex network of regulatory mechanisms, including global regulatory systems and gene specific factors influence α-toxin production. The best investigated global regulatory system is the accessory gene regulator (*agr*), which contributes to the postexponential growth phase re-gulation of a number of unlinked genes. *In vitro*, most exoproteins, including α-toxin, are preferentially produced in the postexponential growth phase, while cell surface proteins are downregulated (1, 2, 34, 37). Two other global regulatory systems, the exoprotein regulator (*xpr*) and the staphylococcal accessory regulator (*sar*), had not yet been comprehensively investigated, but have similar effects on the synthesis of exoproteins as *agr* (16, 17, 29, 61). It has been shown that all three global regulators are essential but not sufficient for α-toxin activation. Supplementary regulatory mechanisms may be involved in α-toxin expression. For instance a separate temporal signal independent of *agr* is required for *hla* but not protein A (*spa*) transcription (66).

*S. aureus* infections are usually treated with antibiotics. It is known that low doses of anti-biotics influence the production of various pathogenicity factors in *Escherichia coli*, *Klebsiella pneumoniae*, *Pseudomonas aeruginosa*, *Proteus mirabilis*, *Streptococcus pyogenes* and others (for review see 43). Several former reports describe an enhancing hemolytic activity caused by subinhibitory concentrations of antibiotics, especially by β-lactams, and it is supposed that these commonly applied antibiotics also elevate the α-toxin production (42, 43, 67). Recently, it has been shown that the β-lactam nafcillin induces α-toxin production *in vitro* and also increases the lethal activity of broth filtrates in a mouse model (36).

In view of the influence of both environmental conditions and subinhibitory concentrations of antibiotics on the expression of the alpha-toxin gene of *S. aureus*, there is a need for an assay system in order to investigate said influence systematically. This need is now satisfied by a chromosomally encoded transcriptional *hla*-*lacZ*-fusion which was constructed and used in several assays.

Our system was proven to be suitable to monitor hla expression and it could be used to investigate the influence of environmental signals on the expression of the α-toxin gene. To our knowledge, this is the first report on a chromosomally integrated gene fusion of a staphylococcal virulence determinant and a reporter gene in S. aureus. Compared with a described transcriptional hla-blaZ fusion on a multicopy plasmid (50), the wild-type gene fusion has the advantage that undesireable multicopy effects which could counteract the regulatory events under question in plasmid systems is excluded. By using a very sensitive assay to detect β-galactosidase activity poor β-galactosidse values obtained by the classical method by Miller (24a) was overcome. The system could also be applied for the investigation of transcriptional regulation of other target genes.

The production of bacterial virulence factors is frequently influenced by various environmental stimuli (20, 27, 45). In this study the environmental regulation of the *hla* gene of *S. aureus,* coding for the α-toxin, a major virulence factor involved in *S. aureus* pathogenesis was in-vestigated. Therefore a transcriptional *hla*-*lacZ* fusion was constructed and integrated into the chromosome of *S. aureus* Wood 46. In consequence, the β-galadosidase production is equi-valent to *hla* mRNA production and could be quantified as an indicator of the *hla* promoter activity. The study revealed that *hla* expression was influenced by changes of growth phase, temperature, O₂-tension, medium and antibiotics.

We measured the *hla* expression during a 24 h growth period. The *hla* expression was weakly detectable during the logarithmic phase, however increased significantly at the end of the log-phase and the beginning of the stationary phase. These results were consistent with pre- vious observations (1, 37, 50) but also indicate that α-toxin is expressed during the log phase on a low level. The growth phase dependent regulation of α-toxin is very complex. It has been shown that at least three global regulators (*agr, sar, xpr*) are involved. Much has been learned in recent years about the agr system, the first described global regulator in *S. aureus*. The *agr* is a polycistronic locus that encode a two-component signal transduction system. The central element of *agr* regulation is a 514 nt RNA molecule, designated RNAIII. RNAIII regulates exoprotein and cell surface protein production of many genes involved in pathogenicity (2, 37, 50) and acts primarily on the level of transcription and in the case of α-toxin also on the level of translation (48, 50). *Agr* negative mutants do not produce many exoproteins and also lack the production of α-toxin (37, 50). Coordinately regulated virulence determinants in bacteria are often controlled by environmental stimuli (27, 45). However, it has recently been shown that RAP, a factor, which is produced and secreted by the bacteria themselves, induces the *agr* system (4). It still remains to be elucidated whether environmental signals influence the pro- duction of RAP. In addition to *agr,* two other global regulatory systems, designated *sar* and *xpr*, which have similar effects on exoprotein and cell surface protein synthesis, were des-cribed (16, 17, 29, 61). It is assumed that these regulators interact with *agr* and modulate *agr* expression (18, 61). Finally it has been demonstrated that a second activatory signal in-dependent of *agr* is required for postexponential regulation of *hla* expression (53). These findings indicate the general multifactorial character of postexponential exoprotein synthesis in *S. aureus* and particulary for α-toxin.

The temperature dependence of *hla*-expression showed some interesting features. Unlike other pathogenic bacteria which express virulence factors mostly at the human body temperature, e.g. *E. coli* P-fimbriae or S-fimbriae (25, 59), or at 30° C e.g. the cholera-toxin of *Vibrio cholerae* (22) or *Yersinia enterocolitica* enterotoxin *Yst* (46), staphylococcal α-toxin is optimally expressed at 42° C. In *S. aureus* little is known about the influence of temperature on the expression of virulence genes. Notable is the fact that both gram positive cell wall and α-toxin itself trigger the production of IL-1 and TNF a, two potent endogenous pyogenics (8, 30, 63). Thus the staphylococcal α-toxin producing strains may be involved in increasing the body temperature and our data show that higher temperature lead to an increase of α-toxin production. These data may indicate the importance of α-toxin in infecions of inner organs or septicemia.

*S. aureus* is a halotolerant bacterium and can be isolated from environments that exert a high level of osmotic stress. However, this organism is also capable to grow in low osmotic con- ditions. In gram negative pathogens the salt concentration affects the expression of virulence factors in various bacteria, such as cholera toxin, adhesins in *Vibrio cholera* (22), or *E. coli* S-fimbriae (59). Here we showed that α-toxin is osmotically regulated. Interestingly, the highest level of *hla* transcription was observed when cultivation occured without NaCl. Similar results were obtained by Sheehan *et al.*(60) who investigated the *S. aureus* epi- dermolytic toxin A (*eta*). We determined a second peak of α-toxin expression at 0.2 M NaCl. For *V. cholera* cholera toxin (22) and *E. coli* S-fimbriae (59) an optimal gene expression was reported below 0.1 M NaCl. This could reflect a specific adaption of *S. aureus* to NaCl, which may be a common component of the habitat of this bacterium. Higher osmolarities leaded to decreased *hla* expression and over 1.6 M no expression was detected. Several groups exa- mined the influence of high osmolarity of staphylococcal enterotoxin A, B, C (SEA, SEB, SEC) (33, 54, 64). Whereas the SEA production was less sensitive to high- osmotic-strength medium, SEB and SEC production was more tightly regulated by osmotic pressure (24, 25). It has been shown that the influence of high osmolarity on *sec* expression is *agr* independent (54). Experiments to elucidate the influence of *agr* on α-toxin expression under high-osmotic-strength conditions are under way.

Another physiological factor found to influence *hla* expression is glucose. The addition of glucose to the growth medium resulted in a decrease of *hla* expression in a concentration dependent manner. Regassa *et al.* (55, 56) have shown that the glucose effect on *hla* expression is a result of two cumultative effects, a decrease of RNA III, the mediator of *agr,* and additionally an *agr* independent event. They reported a 16-fold decrease of *hla* mRNA following cultivation in the presence of 0.1 M glucose. It remains unresolved how glucose influence RNA III and *hla* expression and it is speculated that it occurs at the mRNA level (56). The results of this study showing a 15-fold decrease of the ha expression following cultivation with 0.1 M glucose are consistent with those findings. The glucose effect in *S. aureus* is different from the catabolite repression in *E. coli*, which can be overcome by the addition of exogenous cyclic AMP (65). The addition of cyclic AMP to glucose grown *S. aureus* cultures did not reverse the glucose repression of SEA or SEB production (33, 62).

The production of *hla* showed a strong oxygen dependence. Under anaerobic conditions almost no *hla* expression was detectable. Moreover, the expression of *hla* was induced after cultivation on solid medium. Such an induction was observed for attachment organells, such as flagellae or fimbriae, of gram negative bacteria (5, 59). *S. aureus* is able to colonize a variety of tissues. The organism frequently forms abcesses on the skin. Moreover, after invasion of the skin and mucous memranes, it can enter the blood stream and spread to many organs. *S. aureus* can seed to heart valves, bones, joints, kidneys and other tissues (68, 70). Cultivation on solid medium was shown to enhance α-toxin expression. Our findings implicate an *in vivo* relevance of α-toxin caused necrosis due to *S. aureus* infections. The exact role of α-toxin in manifestation of human diseases caused by *S. aureus* is presently unclear, however, most pathogenic *S. aureus* strains elaborate α-toxin and many human cell types are highly susceptible to the toxin (8). The data presented in this study revealed that α-toxin expression is altered by certain environmental stimuli which may contribute to the *in vivo* virulence of *S. aureus.*

It has been shown that subinhibitory concentrations (MIC) of antimicrobial agents have multiple effects on bacterial cells (43). Several reports observed a direct influence of MIC on the production of pathogenicity factors in both gram positive and gram negative bacteria (for review see 43). The hemolytic ability of *S. aureus* was reported to be enhanced by certain antibiotics, especially by β-lactams (42, 43, 67). While investigating the influence of MICs of antibiotics on transcriptional level, an inductive or repressive influence of several antibiotics on the *hla* expression was found. The β-lactams methicillin and oxacillin dramatically increased *hla* expression, whereas rifampicin and erythromycin repressed *hla* transcription. Other antibiotics, such as vancomycin, fosfomycin and trimethoprim had a weak stimulatary effect. It remains to be elucidated whether the antibiotics directly interact with the promoter or whether they influence regulatory systems such as *agr.* It has recently been shown that nafcillin induces α-toxin production and also increases the lethal activity of broth filtrates in a mouse model (36). It has also been shown that regulatory mechanisms other than *agr* contribute to the induction of α-toxin production by nafcillin.

The finding that low doses of commonly used antibiotics induce α-toxin production may have relevance for serious *S. aureus* infections. Further investigations are required to determine whether antibiotics induce α-toxin production *in vivo* and whether this plays a role in the pathogenesis of *S. aureus* infections.

One embodiment of the invention is a bacterial strain, obtainable by transforming *Staphylococcus aureus* Wood 46 with a hla-lacZ fusion gene using the recombinant plasmid pKO 10 carrying said fusion gene, growing the transformants overnight at about 30°C in the presence of chloramphenicol, plating the transformants onto BHI plates with chloramphenicol, incubating said plates at the non-permissive temperature of 43°C and analyzing chlormphenicol resistant colonies at the non-permissive temperature by Southern hybridization using the cloned *hla*-fragment as a probe upon chromosomal integration of pKO 10 into the promoter region of the *hla*-gene of Wood 46.

Another embodiment is bacterial strain *Staphylococcus aureus* Wood 46-3.

One embodiment of the invention is a Staphylococcus aureus strain containing a reporter gene operatively linked to the promoter of the hla-gene, preferably wherein the reporter gene is the lacZ gene.

A further embodiment of the invention is the use of a bacterial strain to measure the hla promoter activity under different growth conditions, preferably wherein the different growth conditions are selected from the group consisting of length of growth time and phase, temperature, osmolarity, glucose concentration, solid/liquid media, oxygen and concentration of low, subinhibitory concentrations of antibiotics.

Another embodiment of the present invention is vector pKO10.

Another embodiment of the invention is a process for the production of Staphylococcus aureaus Wood 46-3, wherein
a) Staphylococcus aureus Wood 46 is transformed with the recombinant plasmid pKO10 carrying the hla-lacZ fusion gene;
b) growing the transformants overnight at about 30°C in the presence of chloramphenicol;
c) plating the transformants onto BHI plates with chloramphenicol;
d) incubating said plates at the non-permissive temperature of 43°C and
e) analyzing chlormphenicol resistant colonies at the non-permissive temperature by Southern hybridization using the cloned hla-fragment as a probe upon chromosomal integration of pKO10 into the promoter region of the hla-gene of Wood 46.

In the following the invention is described by and without limiting the scope of the invention to the figures, examples and tables.

### LEGENDS TO FIGURES

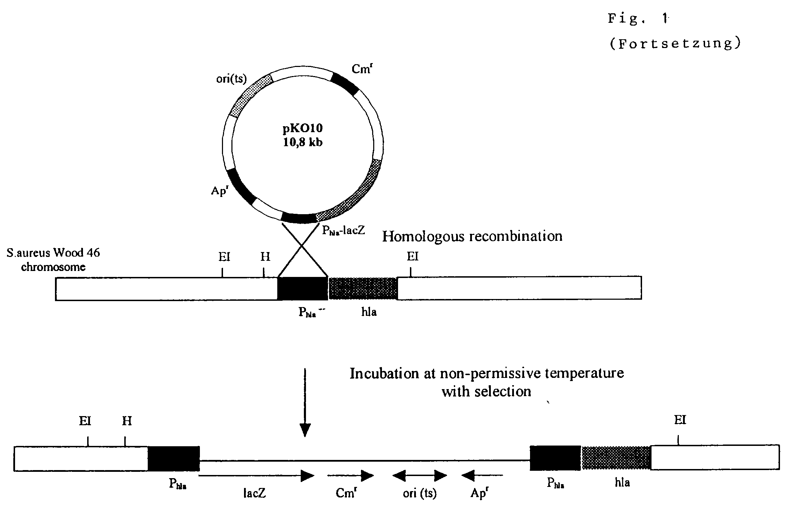

FIG. 1. Strategy for the construction of the chromosomally encoded *hla*-*lacZ* fusion. The *hla* promoter region used to generate the transcriptional fusion was amplified by PCR. *EcoRI* and *BamHI* restriction sites were introduced to fascilate subsequently cloning. Fusion vector pKO 10 was first transformed into *E. coli* MC 4100 cells, following transformation of *S. aureus* RN 4220 and *S. aureus* Wood 46 cells. The homologous recombination was carried out like described in the text. Ampicillin resistance (Ap 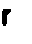 ) is only expressed in gram-negative species, whereas Chloramphenicol resistance (Cm^{r}) is expressed in gram-positive species. Ori (ts) is a gram-positive temperature-sensitive origin of replication from pTV 1 (ts) Restriction site abbreviations: B, *BamHI*; EI, *EcoRI*; EV, *EcoRV*; N, *Nsil*.

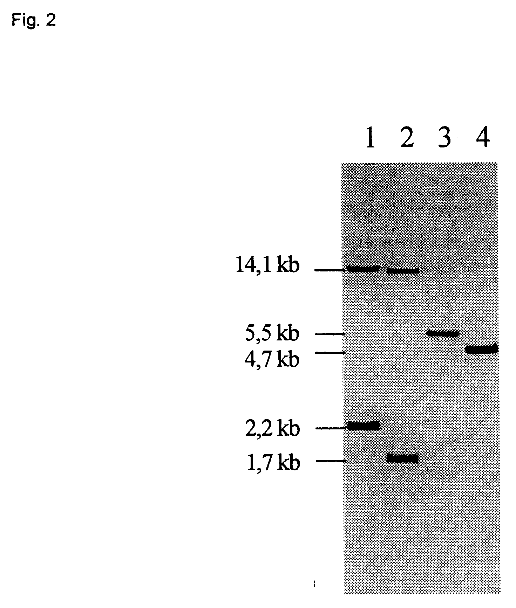

FIG. 2. Southern blot analyses of chromosomal DNA from *S. aureus* wild-type Wood 46 and *hla*-*lacZ* fusion strain Wood 46-3. Chromosomal DNA was cleaved with *EcoRI* (Lane 1 and 3) and *HindIII* (Lane 2 and 4) and probed with cloned 1,1 kb *EcoRI-BamHI* fragment containing *hla* promoter region. DNA was isolated from strain Wood 46 (Lane 3 and 4) and Wood 46-3 (Lane 1 and 2). Sizes in kb are indicated.

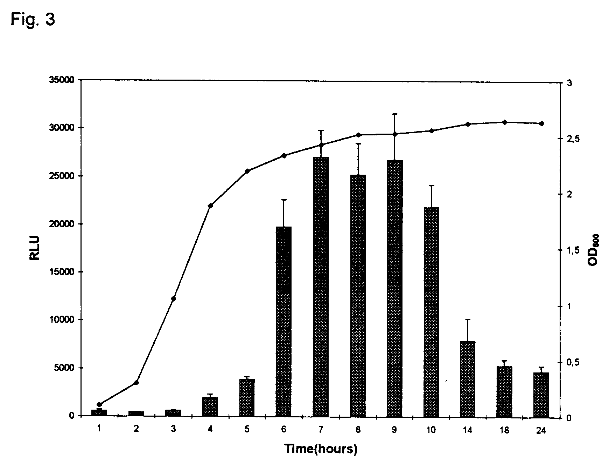

FIG. 3. Kinetics of transcription of a chromosomal *hla*-*lacZ* fusion. β-galactosidase activity indicated by bars is expressed in relative light units (RLU). Assays were performed as described in materials and methods. Line indicates growth as measured by optical density at 600 nm. Mean value h standard deviation of 6 experiments are given.

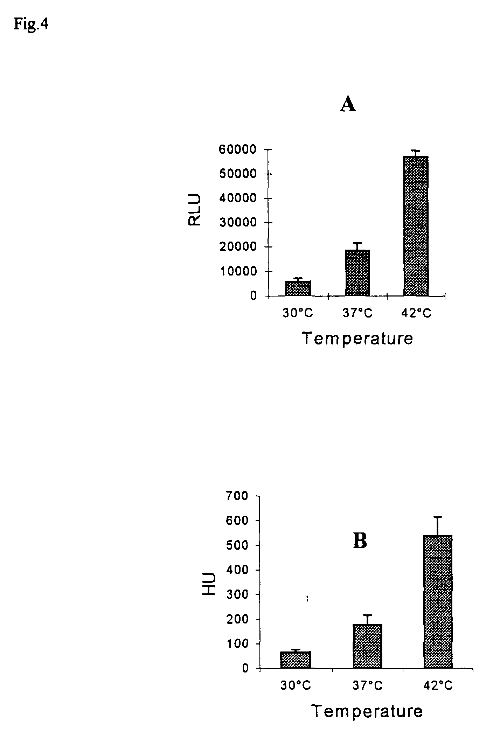

FIG. 4. Temperature dependence of *hla-lacZ* transcription in *S. aureus* Wood 46-3 (A) and of hemolytic activity of Wood 46 (B). β-galactosidase activity is given as relative light units (RLU) and hemolytic activity as hemolytic units (HU). Cultures were grown at different temperatures and assays were performed as described in the text. Data are means ± SD of 6 experiments.

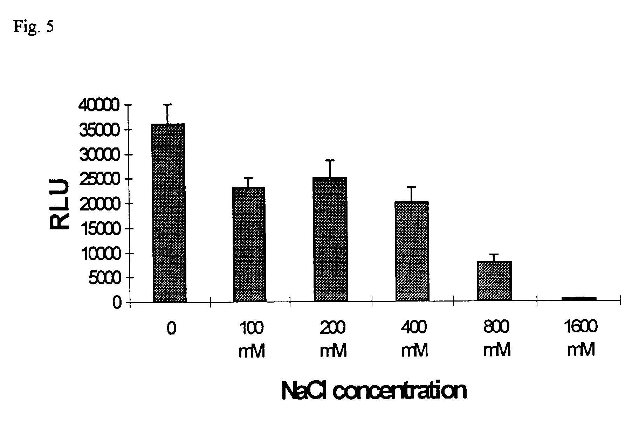

FIG. 5. β-galactosidase production at varying osmolarities given as relative light units (RLU). Cultures were grown as described with varying NaCl concentrations. Data are means ± SD of 3 experiments.

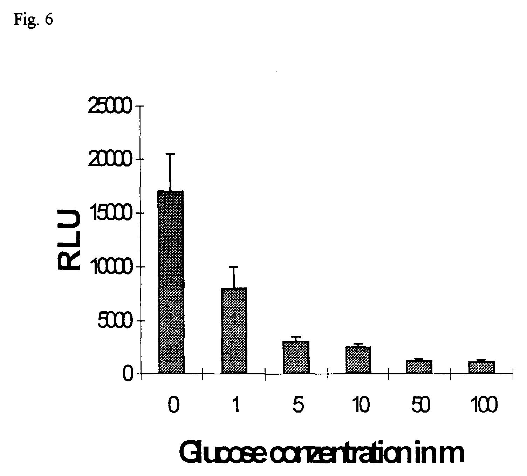

FIG. 6. Effect of glucose on β-galactosidase production indicated as relative light units (RLU). Wood 46-3 cultures grown in LB medium without glucose and with 1 mM, 5 mM, 10 mM, 50 mM and 100 mM respectively. Mean values ± SD of 3 experiments are given.

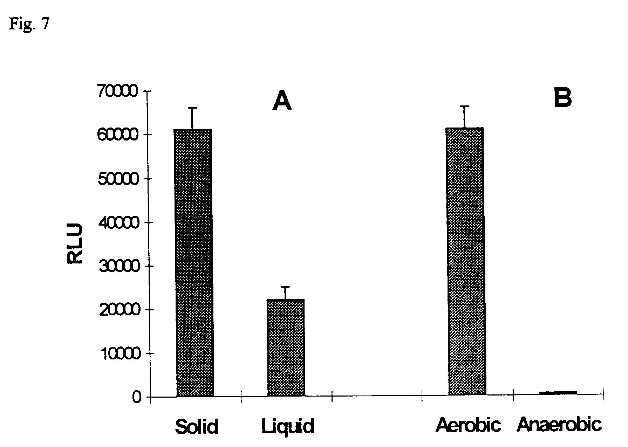

FIG. 7. β-galactosidase production given as relative light units (RLU) by the fusion strain Wood 46-3 grown on solid or liquid medium (A) and under aerobic and anaerobic conditions on solid medium (B) as described in materials and methods. Data are means ± SD of 3 experiments.

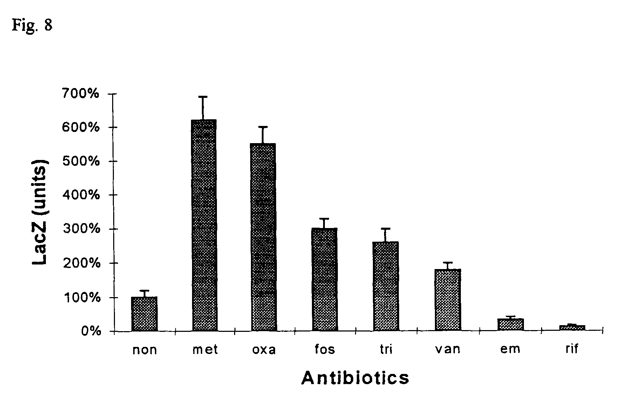

FIG. 8. Influence of ¼ sub-MIC of antibiotics on β-galactosidase (LacZ) production of the fusion strain Wood 46-3 grown in liquid medium up to postexponential phase. LacZ units are given as % values of the LacZ production after growing Wood 46-3 without antibiotics which was set as 100%. Mean ± SD of 5 experiments are given. Abbreviations: met, methicillin; oxa, oxacillin; van, vancomycin; fos, fosfomycin; tri, trimethoprim; rif, rifampicin; em, erythromycin.

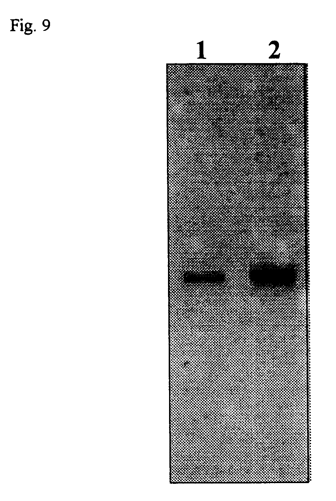

FIG. 9. Northern blot analysis of samples isolated from strain *S. aureus* Wood 46-3 grown without (Lane 1) and with 0,5 µg/ml methicillin (Lane 2). The samples contained the same amount of total cellular RNA. Hybridization was done using a 0,7 kb *Cla I* intragenic *hla* probe.

In the examples the following materials and general methods were used:

**Strains and plasmids*.*** The bacterial strains and plasmids used in this study are listed in Table 1. *S. aureus* Wood 46 is an α-toxin producing strain and was used to introduce a *hla*-*lacZ* gene fusion into the chromosome. RN4220 is a restriction negative mutant o*f S. aureus* 8325-4 and capable for accepting *E. coli* DNA (39). MC4100, a *lac* negative *E. coli* strain (13), was used as host strain in cloning experiments. pDH32M is a *Bacillus subtilis* integration vector, containing a promoterless *lacZ* gene preceded by the *B. subtilis spoVG* ribosome binding site (38). pBT1 (12) is a temperature sensitive shuttle vector and was constructed by ligating a 2,3 kb *Pvull-BamHI* fragment from pBR322 with a 4,0 kb *Pstl* (filled in by Klenow fragment) - *BamHI* fragment of pTV 1 (Ts) (71).

**Media, chemicals and enzymes.** *Escherichia coli* strains were grown in LB broth (57). *Staphylococcus aureus* strains were cultured in either brain heart infusion (BHI) (Difco, Augsburg, Germany) or LB broth. The recombinant *E. coli* and *S. aureus* clones were cultivated under selective antibiotic pressure with either ampicillin (100 µg/ml ) or chlor- amphenicol (10 µg/ml), respectively. Antibiotics were purchased from Sigma, Deisenhofen, Germany. Restriction enzymes, Klenow fragment and T4 DNA ligase were purchased from Pharmacia, Freiburg, Germany. For detection of Lac⁺ colonies, LB plates containing 0.01% 5-bromo-4-chloro-3-indolyl-b-D-galactoside (X-gal) (GERBU, Gaiberg, Germany) were used.

**Bacterial growth conditions.** To study the influence of the growth phase on α-toxin expression, overnight cultures of Wood 46-3 were diluted 1:50 in LB broth and subsequently cultivated in 100 ml flasks at 37°C with shaking for 24 hours. Samples were removed during the first 10 h and after 14 h, 18 h and 24 h. In order to investigate the influence of certain environmental conditions and subinhibitory concentrations of antibiotics on α-toxin pro- duction, overnight cultures were diluted (start OD₆₀₀ ∼0.05) and grown until the post- exponential phase (OD ₆₀₀ 2.4) was reached. For experiments investigating the influence of os- molarity on α-toxin production, LB was modified in respect to its NaCl-concentrations, ranging from 0 M to 1.6 M. Temperature effects were studied at 30° C, 37° C and 42° C. Glucose was added to a final concentration of 0 M to 0.1 M. Subinhibitory concentrations of antibiotics were supplemented as listed in Table 2. Solid cultures were grown overnight on LB agar. Cultivation under anaerobic conditions was done using the anaerobic system Anaerocult (Merck, Darmstadt, Germany).

**Recombinant DNA techniques.** Endonuclease restrictions, ligations, Klenow reactions and electrophoresis were performed as recommended by the manufactures. Isolation and puri- fication of DNA fragments from agarose gels was performed using the Gene-clean-kit by Bio 101, Vista, Calif.. Isolation of plasmid DNA from *E. coli* was carried out by the alkaline method of Birnboim and Doly (10). The same procedure was modified by the addition of 50 µg/ml of lysostaphin (Sigma, Deisenhofen, Germany) in the lysis step to isolate plasmid DNA from *S. aureus*. Plasmid DNA was introduced into competent *E.coli* MC4100 by the CaCl₂ - method (57). *S. aureus* was transformed by electroporation (58). All plasmid constructions were done in *E. coli* and subsequently transferred into the restriction deficient *S. aureus* strain RN 4220 by electroporation prior to transforming *S. aureus* Wood 46.

### Example 1: Construction of plasmids.

As indicated in Fig. 1, the alpha-toxin promoter was amplified from *S. aureus* Wood 46 by PCR by using the primers 5'-GTTTGATATGGAATTCCTGAA TTTTTC-3' (SEQ ID NO.: 1), generating an *Eco RI*-site at the beginning, and 3'-CTACTTTTACTTTTGTGC ATACCTAGGAGTCA-5' (SEQ ID NO.: 2), generating a *BamHI* site at the end, and consecutively cloned into pDH32M creating pKO8. A 4.6 kb *EcoRI-Nsil* fragment of pKO8, containing the trans-criptional fusion of the *hla* promoter and the promoterless *lacZ* gene, was cloned into the *EcoRI-EcoRV* restricted shuttle vector pBT1, generating pKO10. Plasmid pKO3 was con-structed by cloning of a 3.5 kb *Eco RI-Nsi l* fragment of pDH32M, containing the promoterless *lacZ* gene into the *EcoRI-EcoRV* digested shuttle vector pBT1.

### Example 2: Generation of DNA probes and Southern blot analysis.

Chromosomal DNA was prepared from cells grown overnight in BHI by a method of Pospiech and Neumann (53). The protocol was modified by substituting lysozyme by 50 µg/ml lysostaphin . Following its digestion with restriction endonucleases, the resulting fragments were seperated by electrophoresis using a 0.8 % agarose gel. The Southern transfer of the DNA to a Biodyne B membrane (Pall, Portsmouth, U.K.) was performed using a vacuum blot apparatus (Vacu Gene, Pharmacia, Freiburg, Germany) . The gel was submerged successively in depurination (0.25 M HCl), denaturation (0.5M NaOH, 1.5 M NaCl), and neutralization (0.5M Tris-HCl pH 8.0, 1.5 M NaCl) solutions for 8 min and 20xSSC (SSC; 0.15M NaCl, 0.015 M sodium citrate, pH 7.0) for 40 min. The blotted DNA was crosslinked on the filter using a GS Gene Linker UV chamber (Biorad, Richmond, Calif.). For the detection of the *hla* gene the cloned 1.1 kb *Eco RI-BamHI* fragment containing *hla*-promoter was used as a DNA probe. The hybridization was carried out using an ECL direct nucleic acid labelling and detection system from Amersham according to the manufacture's instructions (Amersham, Braunschweig, Germany).

### Example 3: Extraction of RNA and DNA-RNA hybridization.

RNA was prepared by using a RNeasy-kit (Qiagen, Hillen, Germany). For Northern blot analysis samples with the same amount of total cellular RNA, determined by measuring the A₂₆₀, were electrophoresed and transferred to Biodyne B nylon membrane (Pall, Portsmouth, U.K.) as described (3). The probe, a 722 nt intragenic *Clal* fragment (26) from the *hla* gene, was labelled using an ECL- kit from Amersham (Amersham, Braunschweig, Germany) and the hybridization was per- formed according to the manufacture's instructions.

### Example 4: SDS-PAGE and immunoblotting.

SDS-PAGE was performed as described by Laemmli using discontinous 12.5 % acrylamid gels (41). For immunoblot analysis, proteins were transferred to nylon membrane by semidry electroblotting in a graphite chamber according to the method described by Kyhse-Anderson (40). Following the blot of the membranes, blocking occured using 5% bovine serum albumin (BSA) (GERBU, Gaiberg, Germany) in TBS for 1 h. The filters were then incubated for 1 h with a polyclonal anti-alpha-toxin antibody in TBS containing 0.05% Tween 20 (Sigma, Deisenhofen, Germany), following a 1 h-incubation with HRP-conjugated anti-rabbit Antiserum (DAKO, Hamburg, Germany) diluted 1:1000. Blots were developed using 4-chloro-1-naphtol and hydrogen peroxide.

### Example 5: β-Galactosidase tests.

β-Galactosidase assays were performed using a Galacto light ^{TM} chemiluminescent reporter assay system (Tropix, Bedford, Mass.). After cultivation described above bacterial cells were harvested, washed and resuspended in 0.9% NaCl. The cell sus- pension was adjusted to an OD₆₀₀ of 1.0. 0.5 ml of this suspension were centrifuged (8000 rpm, 10 min) and the cell pellet was resuspended in lysis buffer (0.01 M potassium phosphate buffer pH 7.8 ; 0.015M EDTA, 1 % Triton X-100, 50 µg/ml lysostaphin) and incubated at 37°C for 15 min. Following centrifugation (15 min, 13000 rpm), 10 µl of the culture supernatant was used performing a β-galactosidase assay according to the manufacture's instructions. The β-galactosidase activity was measured by a luminometer LB 9501 (Berthold, Wildbad, Germany) with a 300 µl automatic injector and using a 5 second integral.

### Example 6: Assays of hemolytic activity.

After cultivation of *S. aureus* Wood 46-3, the bacteria were pelleted and the supernatant was serielly diluted in 0.15 M NaCl , 0.01 M Tris pH 7.2 containing 0.1% BSA. Aliquots were added to a 1% suspension of washed rabbit blood cells and incubated at 37° C for 30 min as described by Bernheimer (6). The inverse of the tube solution resulting in 50% hemolysis was defined as the number of hemolytic units per milliliter of the test solution.

**Antibiotics.** The antibiotics used are listed in Table 2.

**MIC determinations.** The MIC determination was performed by tube assay DIN 58940, and the results are given in Table 2

**Statistics.** Mean values ± standard deviation were calculated by the method of Cavalli-Sforza (14).

### Example 7: Construction of the wild-type hla-lacZ fusion in S. aureus Wood 46-3.

The construction of the transcriptional fusion strain Wood 46-3 is indicated in Fig. 1. The recombinant plasmid pKO 10, carrying the *hla-lacZ* fusion (see materials and methods) was transformed into *S. aureus* Wood 46. Transformants were grown overnight at 30 °C in the presence of chloramphenicol to generate a population of plasmid-bearing cells. Serial dilutions of this culture were plated onto BHI plates with chloramphenicol and incubated at the non-permissive temperature of 43°C. To determine whether pKO 10 was integrated into the upstream se- quence of the *hla* gene of strain Wood 46, chloramphenicol resistant colonies at the non- permissive temperature were analysed by Southern hybridization using the cloned *hla* fragment as probe. As demonstrated in Fig. 2, Wood 46 wildtype hybridized with a single *Eco RI* fragment (5.5 kb in size) and also one *Hind III* fragment (4.8 kb in size) of the chro-mosomal DNA. The appearence of two *Eco RI*-fragments of 14.1 kb and 2.2 kb and also two *Hind III*-fragments of 13.9 kb and 1.7 kb indicated a site-specific integration of pKO 10 into the promoter region of the *hla* gene of Wood 46.

### Example 8: Influence of the length of growth time and phase on the expression of hla.

To analyse the *hla* transcription in *S. aureus* Wood 46 the constructed *hla-lacZ* fusion was used to monitor gene expression during a growth time of 24 h. The strain was grown in liquid culture and the β-galactosidase activity, indicating *hla*-gene expression, was investigated. At each time, an equal number of bacterial cells (1x10⁹ CFU/ml) was used. As indicated in Fig. 3, *hla* expression was dependent on the growth phase. The rate of transcription of the *hla*-*lacZ* fusion increased significantly during the transition from the exponential to the stationary growth phase. At the early logarythmic phase only a very slight β-galactosidase activity was measured. Up to 3 h the β-galactosidase activity was about 520 RLU and increased slowly during the log-phase (3800 RLU after 5 h). However, at the end of the log-phase and at the early stationary phase the β-galactosidase activity increased from 3800 RLU after 5 h to 19500 RLU after 6 h. During the following four hours the β-galactosidase level increased only slightly and was maintained on a high level. Subsequently (after 14 h and 18 h) a decrease of the β-galactosidase activity was detected, and after 24 h the level reached 4800 RLU.

### Example 9: Influence of the temperature on the expression of the hla gene.

The influence of the temperature on the expression of the *hla-lacZ* in *S. aureus* Wood 46-3 was also determined. As shown in Fig. 4 A, α-toxin production was influenced by temperature. Surprisingly, op- timal expression of β-galactosidase, representing *hla*-expression, was obtained at 42 ° C. A significant increase of the β-galactosidase level was measured after cultivation of Wood 46-3 at 42° C (18400 RLU at 37° C cultivation versus 57100 RLU at 42 ° C). At 30 ° C the β-galactosidase activity was only one third compared to 37°C.

### Example 10:Influence of the temperature on the hemolytic activity of Wood 46.

To compare the temperature dependent *hla* expression obtained with the *hla*-*lacZ* fusion in Wood 46-3 with the hemolytic activity of the parental strain Wood 46, the hemolytic activity of supernatants was measured following growth at 30° C, 37° C and 42° C. As indicated in Fig. 4 B, the hemolytic activity was highest when growth occured at 42° C; 538 HU). Lower temperatures leaded to a decreased hemolytic activity (181 HU at 37° C and 64 HU at 30° C).

### Example 11:Influence of the osmolarity.

In order to examine the influence of osmolarity on *hla* expression strain, Wood 46-3 was grown in LB broth containing different amounts of sodium chloride (see materials and methods). Although *S. aureus* is a halotolerant, the cultures grew more slowly at higher osmotic strength (above 0.2 M). The samples were taken for every osmolarity postexponentially and equal numbers of cells were assayed. As indicated in Fig. 5 the highest level of expression was observed after cultivation in LB lacking NaCl. Cultivation with 0.1 M NaCl leaded to decreased β-galactosidase production, while 0.2 M slightly increased the β-galactosidase production again. Further increasing amounts of NaCl decreased the *hla* dependent β-galactosidase production. At 0.8 M only 7800 RLU were measured and concentrations of NaCl above 1.6 M were inhibitory for *hla* expression.

### Example 12:Influence of glucose.

The effect of varying concentrations of glucose in the growth medium on the expression of the *hla-lacZ* fusion was investigated. Previous studies have shown that glucose had a decreasing effect of α-toxin production. We demonstrated that already at very low concentrations of glucose (0.001 M) the β-galactosidase production of Wood 46-3 decreased from 18600 RLU, obtained during cultivation without glucose, to 8200 RLU when 0.001 M glucose were added to the culture medium (Fig. 6). Further increase of the glucose concentration to 0.01 M resulted in a decrease of the level of β-galactosidase activity to 2800 RLU. Higher glucose concentations of up to 0.1 M decreased the β-galactosidase level to 1200 RLU.

### Example 13:Influence of solid and liquid media.

The influence of solid and liquid media on the toxin production was tested. Strain Wood 46-3, wich carries the *hla-lacZ* fusion on its chro-mosome, was comperatively grown overnight on solid medium as well as in a liquid culture. It could be shown that solid grown cultures had threefold more β-galactosidase activity than the liquid cultures (Fig.7 A).

### Example 14:Influence of Oxygen.

Cultures of *S. aureus* Wood 46-3 were grown aerobicly for 18 h on solid medium as well as anaerobically at 37 ° C. As shown in Fig. 7 B, oxygen had a sig-nificant effect on β-galactosidase expression. Anaerobic growth repressed β-galactosidase ac- tivity to almost zero.

### Example 15:Effects of low, subinhibitory concentrations of antibiotics on expression of the alpha-toxin gene.

The *S. aureus* strain Wood 46-3, carrying a transcriptionally *hla-lacZ* fusion on its chromosome was used to determine the influence of subinhibitory concentrations of antibiotics on the *hla* expression. In consequence of the construction of the *hla-lacZ* gene fusion, a chloramphenicol resistance gene was introduced into the chromosome of strain Wood 46. First, the minimal inhibitory concentration (MIC) of the antibiotics was determined. Wood 46-3 was susceptible to all tested antibiotics except of chloramphenicol. In order to test the influence of subinhibitory concentrations of antibiotics mentioned in Tab. 2 on the β-galactosidase activity, the *hla-lacZ* containing strain was grown in LB broth with a quarter of the MIC values until they reached the postexponential growth phase. As a control we used *S. aureus* strain Wood 46-3 grown without antibiotic. This value was set as 100 %. As shown in Fig. 8, the β-lactam antibiotics methicillin and oxacillin had a dramatic effect on the β-galactosidase production. A sixfold increase of the β-galactosidase activity was obtained following cultivation in medium containing methicillin compared to cultivation in medium without antibiotic (28000 RLU grown without antibiotic and 167000 RLU grown with methicillin). Oxacillin exhibited comparable β-galactosidase values (158000 RLU versus 28000 RLU). In order to verify the results obtained with the *hla-lacZ* gene fusion in Wood 46-3 a Northern blot analysis of samples prepared from Wood 46 WT cultures grown without and with 0.5 µg/ml methicillin was performed. A greater amount of hla mRNA was detected by Northern hybridization when cultures were grown in methicillin containing medium than when they were grown without this antibiotic (Fig. 9).

Other substances had a weaker (trimethoprim 46000 RLU, fosfomycin 72000 RLU) or a moderate (vancomycin 37000 RLU) stimulatory effect on β-galactosidase expression. However, rifampicin and erythromycin have shown a strong negative effect on the β-galactosidase expression. A reduction of the β-galactosidase values from 28000 RLU to 3300 RLU (rifampicin) and to 7800 RLU (erythromycin) was measured.

### REFERENCES

1. Abbas-Ali, B., and G. Coleman. 1977. The characteristics of extracellular protein secretion by *Staphylococcus aureus* (Wood 46) and their relationship to the regulation of α-toxin formation. J. Gen. Microbiol. 99:277-282.
2. Arvidson, S., L. Janzon, and S. Lofdahl. 1990. The role of the d-lysin gene (*hld*) in the *agr*-dependent regulation of exoprotein synthesis in *Staphylococcus aureus*, p. 419-431. *In* R. P. Novick (ed.), Molecular biology of the staphylococci. VCH Publishers Inc., New York.
3. Ausubel, F. M., R. Brent, R. E. Kingston, D. A. Moore, J. G. Seidman, J. A. Smith, and K. Strahl. 1987. Current protocols in molecular biology, Vol. 4, John Wiley & Sons Inc., New York.
4. Balaban, N., and R. P. Novick. 1995. Autocrine regulation of toxin synthesis by *Staphylococcus aureus*. Proc. Natl. Acad. Sci. USA 92:1619-1623.
5. Belas, R., M. Simon, and M. Silverman. 1986. Regulation of lateral flagella gene transcription in *Vibrio parahaemolyticus*. J. Bacteriol. 167:210-218.
6. Bernheimer, A. W. Assay of hemolytic toxins. Methods Enzymol. 165: 213-217.
7. Bhakdi, S., and J. Tranum-Jensen. 1991. Alpha-toxin of *Staphylococcus aureus*. Microbiol. Rev. 55:733-751.
8. Bhakdi, S., M. Muhly, S. Korom, and F. Hugo. 1989. Release of interleukin-1 associated with potent cytocidal action of staphylococcal α-toxin on human monocytes. Infec. Immun. 57:3512-3519.
9. Bhakdi, S., M. Muhly, U. Mannhardt, K. Klapptek, C. Müller-Eckhardt, and L. Roka. 1988. Staphylococcal α-toxin promotes blood coagulation via attack on human platelets. J.Exp. Med. 168:527-542.
10. Birnboim, H. C., and J. Doly. 1979. A rapid alkaline extraction procedure for screening recombinant plasmid DNA. Nucleic acids Res. 7:1513-1522.
11. Bramley, A. J., A. H. Patel, M. O 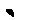 Reilly, R. Foster, and T. J. Foster. 1989. Roles of alpha-toxin and beta-toxin in virulence of *Staphylococcus aureus* for the mouse mammary gland. Infec. Immun. 57:2489-2494.
12. Brückner, R. personal communication.
13. Casadaban, M. J., Chou, J., and S. N. Cohen. 1980. *In vitro* gene fusions that join an enzymatically active β-galactosidase segment to amino-terminal fragments of exogenous proteins: *Escherichia coli* plasmid vectors for the detection and cloning of translational initial signals. J. Bacteriol. 143:971-980.
14. Cavalli-Sforza, L. 1969. Biometrie. Grundzüge biologisch-medizinischer Statistik. Fischer-Verlag, Jena, FRG.
15. Chen, Y., and A. Zychlinsky. 1994. Apoptosis induced by bacterial pathogens. Microbiol. Path. 17:203-212.
16. Cheung, A. L., J. M. Koomey, C. A. Butler, S. J. Projan, and V. A. Fischetti. 1992. Regulation of exoprotein expression in *Staphylococcus aureus* by a locus (*sar*) distinct from *agr*. Proc. Natl. Acad. Sci. USA. 89:6462-6466.
17. Cheung, A. L., and P. Ying. 1994. Regulation of a- and b-hemolysins by the *sar* locus of *Staphylococcus aureus*. J. Bacteriol. 176:580-585.
18. Cheung, A. L., and S. J. Projan. 1994. Cloning and sequencing of *sarA* of *Staphylococcus aureus*, a gene required for the expression of *agr*. J. Bacteriol. 176:4168-4172.
19. Compagnone-Post, P., U. Malyankar, and S.A. Khan. 1991. Role of host factors in the regulation of the enterotoxin B gene. J. Bacteriol. 173:1827-1830.
20. Dorman, C. J. 1991. DNA supercoiling and environmental regulation of gene expression in pathogenic bacteria. Infect. Immun. 59:745-749.
21. Duncan, J. L., and G. J. Cho. 1972. Production of staphylococcal alpha toxin. II. Glucose repression of toxin formation. Infect. Immun. 6:689-694.
22. Gardel, C. L., and J. J. Mekalanos. 1994. Regulation of cholera toxin by temperature, pH, and osmolarity. Meth. Enzymol. 235:517-526.
23. Genigeorgis, C., and W. W. Sadler. 1966. Effect of sodium chloride and pH on enterotoxin B production. J. Bacteriol. 92:1383-1387.
24. Genigeorgis, C., M. S. Foda, A. Mantis, and W. W. Sadler. 1971. Effect of sodium chloride and pH on enterotoxin C production. Appl. Microbiol. 21:862-866.
25. Göransson, M., and B. E. Uhlin. 1984. Environmental temperature regulates transcription of a virulence pili operon in *E. coli*. EMBO J. 3:2885-2888.
26. Gray, S., and M. Kehoe. 1984. Primary sequence of the α-toxin gene from *Staphylococcus aureus* Wood 46. Infect. Immun. 46:615-618.
27. Gross, R. 1993. Signal transduction and virulence regulation in human and animal pathogens. FEMS Microbiol. Rev. 104:301-326.
28. Hacker, J., M. Ott, and H. Hof. 1993. Effects of low, subinhibitory concentrations of antibiotics on expression of virulence gene cluster of pathogenic Escherichia coli by using a wild-type gene fusion. Inter. J. Antimicrobiol. Agents. 2:263-270.
29. Hart, M. E., M. S. Smeltzer, and J. J. Iandolo. 1993. The extracellular protein regulator (*xpr*) affects exoprotein and *agr* mRNA levels in Staphylococcus aureus. J. Bacteriol. 175:7875-7889.
30. Heumann, D., C. Barras, A. Severin, M. P. Glauser, and A. Tomasz. 1994. Gram-positive cell walls stimulate synthesis of tumor necrosis factor alpha and interleukin-6 by human monocytes. Infect. Immun. 62:2715-2721.
31. Hildebrand, A., M. Roth, and S. Bhakdi. 1991. *Staphylococcus aureus* α-toxin: dual mechanisms of binding to target cells. J. Biol. Chem. 266:17195-17200.
32. Iandolo, I. I. 1989. Genetic analysis of extracellular toxins of *Staphylococcus aureus*. Annu. Rev. Microbiol. 43:375-402.
33. Iandolo, J. J., and W. J. Shafer. 1977. Regulation of staphylococcal enterotoxin B. Infect. Immun. 16:610-616.
34. Janzon, L., and S. Arvidson. 1990. The role of the d-lysin gene (*hld*) in the regulation of virulence genes by the accessory gene regulator (*agr*) in *Staphylococcus aureus*. EMBO J. 9:1391-1399.
35. Kehoe, M., J. Duncan, T. Foster, N. Fairweather, and G. Dougan. 1983. Cloning, expression, and mapping of the *Staphylococcus aureus* alpha-hemolysin determinant in *Escherichia coli* K-12. Infect. Immun. 41:1105-1111.
36. Kernodle, D. S., P. A. Mc Graw, N. L. Barg, B. E. Menzies, R. K. R. Voldari, and S. Harshman. 1995. Growth of *Staphylococcus aureus* with nafcillin *in vitro* induces α-toxin production and increases the lethal activity of sterile broth filtrates in a murine model. J. Infect. Dis. 172:410-419.
37. Kornblum, J., B. N. Kreiswirth, S. J. Projan, H. Ross, and R. P. Novick. 1990. *Agr.* a polycistronic locus regulating exoprotein synthesis in Staphylococcus aureus, p. 373-402*. In* R. P. Novick (ed.), Molecular biology of the staphylococci. VCH Publishers Inc., New York.
38. Kraus, A., C. Hueck, D. Gärtner, and W. Hillen. 1994. Catabolite repression of the *Bacillus subtilis xyl* operon involves a cis element functional in the context of an unrelated sequence, and glucose exerts additional *xylR*-dependent repression. J. Bacteriol. 176:1738-1745.
39. Kreiswirth, B. N., S. Lofdahl, M. J. Betley, M. O' Reilly, P. M. Schlievert, M. S. Bergdoll, and R. P. Novick. 1983. The toxic shock syndrome exotoxin structural gene is not detectably transmitted by a prophage. Nature (London) 305:709-712.
40. Kyhse-Anderson, J. 1984. Electroblotting of multiple gels: a simple apparatus without buffer tank for rapid transfer of proteins from polyacrylamide to nitrocellulose. J. Biochem. Biophys. Methods 10:203-209.
41. Laemmli, U. K. 1970. Cleavage of structural proteins during the assembly of the head of bacteriophage T4. Nature (London) 227:680-685.
42. Lorian, V. 1971. Effect of antibiotics on staphylococcal haemolysin production. Appl. Microbiol. 22:106-109.
43. Lorian, V., and G. C. Gemmel. 1991. Effect of low antibiotic concentrations on bacteria: effects on ultrastructure, virulence, and susceptibility to immunodefenses. p. 493-555. *In* V. Lorian (ed.), Antibiotics in laboratory medicine. Williams & Wilkins, Baltimore.
44. Mc Cartney, C., and J. P. Arbuthnott. 1978. Mode of action of membrane-damaging toxins produced by staphylococci, p.89-122. *In* J. Jeljaszewicz and T. Waldstrom (ed.), Bacterial toxins and cell membranes. Academic Press, London.
45. Mekalanos, J. J. 1992. Environmental signals controlling expression of virulence determinants in bacteria. J. Bacteriol. 174:1-7.
46. Mikulskis, A. V., I. Delor, V. H. Thi, and G. R. Cornelis. 1994. Regulation of the *Yersinia enterocolitica enterotoxin yst* gene. Influence of growth phase, temperature, osmolarity, pH and bacterial host factors. Mol. Microbiol. 14:905-915.
47. Miller, V. L., and J. J. Mekalanos. 1988. A novel suicide vector and its use in construction of insertion mutations: osmoregulation of outer membrane proteins and virulence determinants in *Vibrio cholerae requires toxR.* J. Bacteriol. 170:2575-2583.
47a.Miller, J.H. 1972. Experiments in molecular genetics. Cold Spring Harbor Laboratory, Cold Spring Harbor, N.Y.
48. Morfeldt, E., D. Taylor, A. von Gabain and S. Arvidson. 1995. Activation of alpha-toxin translation in *Staphylococcus aureus* by the trans-encoded antisense RNA, RNA III. EMBO J. 14:4569-4577.
49. Musher, D. M., and S. O. Mc Kenzie. 1977. Infections due to *Staphylococcus aureus*. Medicine 56:383-409.
50. Novick, R. P., H. F. Ross, S. J. Projan, J. Kornblum, B. Kreiswirth, and S. Moghazeh. 1993. Synthesis of staphylococcal virulence factors is controlled by a regulatory RNA molecule. EMBO J. 12:3967-3975
51. O 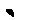 Reilly, M., J. C. S. Azavedo, S. Kennedy, and T. J. Foster. 1986. Inactivation of the alpha-haemolysin gene of *Staphylococcus aureus* 8325-5 by site directed mutagenesis and studies on the expression of its haemolysins. Microb. Pathog. 1:125-138.
52. Patel, A. H., P. Nowlan, E. D. Weavers, and T. Foster. 1987. Virulence of protein A-deficient and alpha-toxin-deficient mutants of *Staphylococcus aureus* isolated by allele replacement. Infec. Immun. 55:3103-3110.
53. Pospiech, A. and B. Neumann. 1995. A versatile quick-prep of genomic DNA from gram-positive bacteria. TIG 11:217-218.
54. Regassa, L. B., J. L. Couch, and M. J. Betley. 1991. Steady-state staphylococcal enterotoxin type C mRNA is affected by a product of the accessory gene regulator (*agr*) and by glucose. Infect. Immun. 59:955-962.
55. Regassa, L. B., and M. J. Betley. 1993. High sodium chloride concentrations inhibit staphylococcal enterotoxin C gene (*sec*) expression at the level of *sec* mRNA. Infect. Immun. 61:1581-1585.
56. Regassa, L. B., R. P. Novick and M. J. Betley. 1992. Glucose and nonmaintained pH decrease expression of the accessory gene regulator (*agr*) in *Staphylococcus aureus*. Infect. Immun. 60:3381-3388.
57. Sambrook, J., E. F. Fritsch and T. Maniatis. 1989. Molecular cloning: a laboratory manual, 2nd ed. Cold Spring Harbor Laboratory, Cold Spring Harbor, N.Y.
58. Schenk, S., and R. A. Laddaga. 1992. Improved method for electroporation of *Staphylococcus aureus*. FEMS Microbiol. Lett. 94:133-138.
59. Schmoll, T., M. Ott, B. Oudega, and J. Hacker. 1990. Use of a wild-type gene fusion to determine the influence of environmental conditions on expression of the S fimbrial adhesin in an Escherichia coli pathogen. J. Bacteriol. 172:5103-5111.
60. Sheehan, B. J., T. J. Foster, C. J. Dorman, S. Park, and G. S. A. B. Stewart. 1992. Osmotic and growth-phase dependent regulation of the *eta* gene of Staphylococcus aureus: a role for DNA supercoiling. Mol. Gen. Genet. 232:49-57.
61. Smeltzer, M. S., M. E. Hart, and J. J. Iandolo. 1993. Phenotypic characterization of *xpr*, a global regulator of extracellular virulence factors in Staphylococcus aureus. Infect. Immun. 61:919-925.
62. Smith, J. L., M. M. Bencivengo, R. L. Buchanan, and C. A. Kunsch. 1986. Enterotoxin A production in *Staphylococcus aureus*: inhibition by glucose. Arch. Microbiol. 144:131-136.
63. Stout, R. D., Y. Li, A. R. Miller, and D. W. Lambe, Jr. 1994. Staphylococcal glycocalyx activates macrophage prostaglandin E₂ and interleukin 1 production and modulates tumor necrosis factor alpha and nitric oxide production. Infect. Immun. 62:4160-4166.
64. Troller, J. A., and J. V. Stinson. 1978. Influence of water on the production of extracellular enzymes by *Staphylococcus aureus*. Appl. Environ. Microbiol. 35:521-526.
65. Ullmann, A., and A. Danchin. 1983. Role of cyclic AMP in bacteria. Adv. Cyclic. Nucleotide Res. 15:1-53.
66. Vandenesch, F., J. Kornblum, and R. P. Novick. 1991. A temporal signal, independent of *agr*, is required for *hla* but not *spa* transcription in *Staphylococcus aureus*. J. Bacteriol. 173:6313-6320.
67. Vymola, F., and D. Lochman. 1974. Effect of antibiotics on *Staphylococcus-haemolysin* production. J. Hyg. Epidemiol. Microbiol. Immunol. 18:281-284.
68. Waldvogel, F. A. 1990. *Staphylococcus aureus* (including toxic shock syndrome), p. 1489-1510. *In* G. L. Mandell, R. G. Douglas, and J. E. Bennet (ed.), Principles and practice of infectious diseases. Churchill Livingstone, New York.
69. Walev, I., E. Martin, D. Jonas, M. Mohamadzadeh, W. Müller-Klieser, L. Kunz, and S. Bhakdi. 1993. Staphylococcal alpha-toxin kills human keratinocytes by permeabilizing the plasma membrane for monovalent ions. Infec. Immun. 61:4972-4979.
70. Wheat, L. J., K. B. Kohler, and A. White. 1983. Diagnosis and management of deep seated infection. p.121-148. *In*: C. S. F. Easmon, and C. Adlam, (ed.), Staphylococci and staphylococcal infections. New York, Academic Press.
71 . Youngman, P. 1987. Plasmid vectors for recovering and exploiting Tn917 transpositions in *Bacillus* and other gram-positive bacteria. p. 79-105. *In*: K.G. Hardy (ed.) Plasmids: A practical approach. IRL Press, Oxford Washington D.C.

**TABLE 1**

| Bacterial strains and plasmids. | | |
|---|---|---|
| Strain or Plasmid | Description | Source or Reference |
| Strains | | |
| *S. aureus* | | |
| Wood 46 | High level production of α-toxin | (35) |
| RN4220 | Derivative of 8325-4, efficient acceptor of *E*.*coli* DNA | (39) |
| Wood 46-3 | Derivative of Wood 46 with *hla-lacZ* fusion | this work |

| *E.coli* | | |
|---|---|---|
| MC4100 | F⁻,araD139, D(argF⁻,lac) U196, rspL150, relA1, deoC1, ptsF25, rbsR, flbB5301 | (13) |

| Plasmids | | |
|---|---|---|
| pDH32M | Integration plasmid for *B.subtilis* | (38), available from Prof. Hinnen and Dr. Krans (University of Erlangen) |
| pBT1 | containing promoterless *lacZ*, Ap 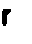 , Cm 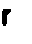 | (12), available from Dr. Brückner (University of Tübingen) |
| pKO3 | *S.aureus-E.coli* shuttle vector containing | this work |
| pKO8 | PE 194 (Ts) and col E1 replicons, Ap 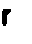 , Cm 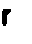 | this work |
| pKO10 | pBT 1 containing promoterless *lacZ* gene, Ap 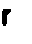 , Cm 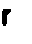 | this work |
| | pDH32M containing transcriptional *hla*-*lacZ* fusion, Ap 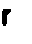 , Cm 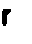 | |
| | pBT1 containing the *hla* promoter transcriptionally fused to *lacZ*, Ap 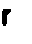 , Cm 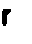 | |

**TABLE 2**

| Antibiotics used in this study. | | |
|---|---|---|
| | Antibiotica (source) | MIC (µg/ml for strain Wood 46-3 |
| 1 | Methicillin (Sigma, Deisenhofen, Germany) | 2 |
| 2 | Oxacillin ( Sigma, Deisenhofen, Germany) | 0,25 |
| 3 | Fosfomycin (Madaus, Köln, Germany) | 0,25 |
| 4 | Trimethoprim (Sigma, Deisenhofen, | 0,25 |
| 5 | Germany) | 1 |
| 6 | Vancomycin (Sigma, Deisenhofen, | 1 |
| 7 | Germany) Erythromycin (Sigma, Deisenhofen, Germany | 0,008 |
| | Rifampicin (Sigma, Deisenhofen, Germany) | |

## Claims

1. Staphylococcus aureus strain containing an reporter gene operatively linked to the promoter of the hla-gene.

2. Staphylococcus aureaus strain according to claim 1, wherein the reporter gene is the lacZ gene.

3. Bacterial strain, obtainable by transforming Staphylococcus aureus Wood 46 with a hla-lacZ fusion gene using the recombinant plasmid pkO10 carrying said fusion gene, growing the transformants overnight at about 30°C in the presence of chloramphenicol, plating the transformants onto BHI plates with chloramphenicol, incubating said plates at the non-permessive temperature of 43°C and analyzing chlormphenicol resistant colonies at the non-permessive temperature by southern hybridization using the cloned hla-fragment as a probe upon chromosomal integration of pkO10 into the promoter region of the hla-gene of wood 46.

4. Bacterial strain Staphylococcus aureus Wood 46-3.

5. Use of a bacterial strain according to any of the preceeding claims to measure the hla promoter activity under different growth conditions.

6. Use of a bacterial strain according to claim 5, wherein the different growth conditions are selected from the group consisting of length of growth time and phase, temperature, osmolarity, glucose concentration, solid/liquid media, oxygen concentration, and concentration of low, subinhibitory concentrations of antibiotics.

7. Vector pkO10.

8. Process for the production of Staphylococcus aureaus Wood 46-3, wherein
a) Staphylococcus aureus Wood 46 is transformed with the recombinant plasmid pKO10 carrying the hla-lacZ fusion gene;
b) growing the transformants overnight at about 30°C in the presence of chloramphenicol;
c) plating the transformants onto BHI plates with chloramphenicol;
d) incubating said plates at the non-permissive temperature of 43°C and
e) analyzing chlormphenicol resistant colonies at the non-permissive temperature by Southern hybridization using the cloned hla-fragment as a probe upon chromosomal integration of pKO10 into the promoter region of the hla-gene of Wood 46.


SEQUENCE LISTING

(1) GENERAL INFORMATION:

(i) APPLICANT:
(A) NAME: Hoechst Aktiengesellschaft
(B) STREET: -
(C) CITY: Frankfurt
(D) STATE: -
(E) COUNTRY: Deutschland
(F) POSTAL CODE (ZIP): 65926
(G) TELEPHONE: 069-305-3005
(H) TELEFAX: 069-35-7175
(I) TELEX: -

(ii) TITLE OF INVENTION: Expression of the Alpha-Toxin Gene (hla) of
Staphylococcus aureus using a chromosomally encoded
hla-lacZ gene fusion

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION: 1..27

(xi) SEQUENCE DESCRIPTION: SEQ ID NO: 1:

GTTTGATATG GAATTCCTGA ATTTTTC 27

(2) INFORMATION FOR SEQ ID NO: 2:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA (genomic)

(ix) FEATURE:

(A) NAME/KEY: exon
(B) LOCATION: 1..32

(xi) SEQUENCE DESCRIPTION: SEQ ID NO: 2:

CTACTTTTAC TTTTGTGCAT ACCTAGGAGT CA 32